# EUROPEAN PATENT APPLICATION

(11) **EP 1 066 852 A1**
(43) Date of publication of application: **10.01.2001**
(21) Application number: 00202322.4
(22) Date of filing: 03.07.2000
(51) Int. Cl.: A61M 31/00

(54) **Cannula for medicinal ointments.**

(30) Priority: 05.07.1999 IT MI991479
(71) Applicant: INGE S.p.A., 20024 Garbagnate Milanese (IT)
(72) Inventor: Nobbio, Alessio, 20123 Milan (IT)
(74) Representative: Parisi, Luigi

(57) **Abstract**

A cannula (11) for medicinal ointments (30) comprising a cylinder (12), capable of containing a medicinal product such as a vaginal and/or anal ointment, and a piston (13) moved by a rod (14) inside a cylinder (12), where the rod (14) is connected to the piston (13) in a detachable form.

## Description

This invention refers to a cannula for medicinal ointments, in particular of a type used for applying vaginal and/or anal ointments.

At present the confections of medicinal products for vaginal or anal uses normally include a container of the ointment and one or more cannulas used for applying the ointment.

The traditional cannulas comprise in fact a collecting cylinder with a sliding internal cylinder firmly connected to an actuating rod.

The cylinder usually offers a threaded extremity which allows associating it by a threading motion with a container or small tube of the medicinal ointment.

The opposite extremity is perforated to allow the sliding of an actuating rod and to allow a proper usage of the cannula, at first by withdrawing the rod to allow admitting the medicinal from the tube and therefore, once the tube has been detached, by expelling the ointment from the threaded extremity, under the pushing action of the flat piston surface, when the latter is made to slide in a direction opposite to that of admitting the product, by the aid of the actuating rod.

For this purpose, the piston may eventually be profiled and offer a larger diameter with respect to the diameter of the actuating rod and essentially matching the internal diameter of the cylinder, so as to slide along the internal cylinder walls with a certain friction and to ensure a sealing action of the coupling during the axial motion of the rod, while applying the product, as well as a pushing action toward the outside of most of the product which has been previously introduced.

These known cannulas offer however a number of drawbacks, such as construction problems and basically high production costs, because of the particular profiling of the piston required at the extremity of the actuating rod.

For hygienic reason, it is moreover an absolute must to provide for the production of disposable cylinders and rods, which leads to a considerable consumption of material and a substantial increase in costs, depending on the number of applications required.

The purpose of this invention is to eliminate the drawbacks mentioned above, by producing a cannula for medicinal ointments capable of facilitating the operation of filling the dispensing cylinder with specific products, such as vaginal or anal ointments, while at the same time ensuring some reduced overall cannula production costs compared to those of the known art.

These and other purposes according to this invention are achieved by producing a cannula for medicinal ointments according to claim 1, which is being referred to for brevity.

According to the invention, it is advantageously possible to limit the consumption of the disposable product to the utmost at every application of the product, while consequently reducing the overall production costs of the medicinal ointments.

Further characteristics and advantages of a cannula for medicinal ointments according to this invention will become more clearly evident from the following description, offered for exemplifying and non-limiting purposes with reference to the simplified enclosed drawings, in which:
- Figure 1 shows a first embodiment of the medical cylinder for cannulas for medicinal ointments according to the invention,
- Figure 2 depicts the cylinder shown in Figure 1, while being filled with the medicinal ointment,
- Figure 3 shows an actuating rod usable for applying the medicinal ointments according to this invention,
- Figure 4 shows an enlarged and partially sectionalized detail relating to the Figures 1 and 2,
- Figure 5 is an exploded, enlarged and partially sectionalized view of an alternative embodiment of a cannula for medicinal products according to this invention,
- Figure 6 is a prospective view of a confection containing a tube of medicinal ointment, a series of medical cylinders and an actuating rod, according to this invention.

With reference to the mentioned figures, a cannula for medicinal ointments is shown in its overall form by the number 11.

The cannula 11 comprises a cylinder 12, open at its two extremities and containing a sliding pusher piston 13 which may be actuated by a rod 14 by a user from the outside.

A first extremity 12' of the cylinder 12 is threaded in 15 so as to allow associating it with a tube or container 16 for an ointment, cream or other medicinal product 30 and for introducing the product 30 into the cylinder 12 of the cannula 11.

In particular, the cannula 11 is used for dispensing vaginal and/or anal ointments.

An area of the cylinder 12 corresponding to the threaded section 15 is fitted with an undercut 17 acting as a stop for the piston 13, which may be held in this position prior to introducing the medicinal ointment 30 into the cannula 11.

The undercut 17 is formed by a profiled ring inside the cylinder 12, extending along at least one turn of the latter's circumference and engaging with an upper ring 22 of the pushing piston 13.

The opposite extremity 12'' of the cylinder 12 may provide a hole 18 for introducing the actuating rod 14 of the piston 13.

Next to this extremity 12'' the cylinder 12 also offers, for a certain length of its internal surface, a diameter larger than that of the rest of the cylinder, and is fitted, precisely next to this change of diameter, with a stop 19 holding back the piston 13, to as to prevent it from slipping out of the small tube 12 while moving the cannula 11.

At the point of the stop 19, the cylinder 12 is also fitted with small air venting gaps 20, necessary for a proper filling of the cannula 11.

The pushing piston 13 has an essentially cylindrical shape and includes an upper protruding ring 22 which ensures the sealing action of the coupling between the piston 13 and the cylinder 12 while moving the pushing piston 13 inside the cannula 11.

Finally, the piston 13 is preferably manufactured from a sliding, low-friction plastic material to facilitate the internal sliding of the cannula 11 and therefore the filling of the cylinder 12 and the dispensing of the product.

The operation of the cannula for the medicinal ointments according to this invention is substantially as follows.

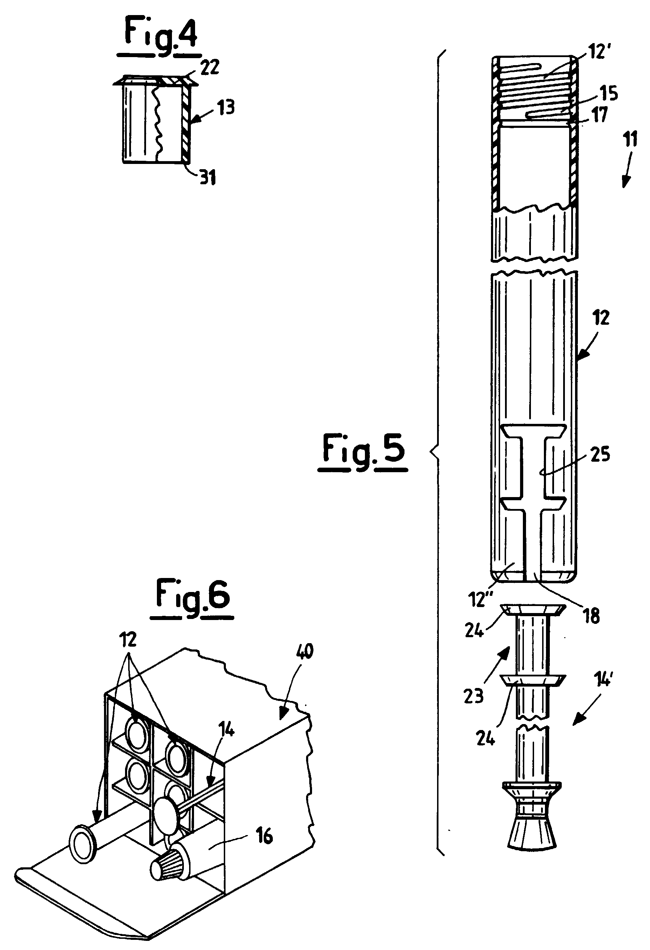

Thanks to the innovative concept of this invention, the confections of medicinal ointments 30 such as those illustrated in Figure 6 and indicated by the reference number 40 may comprise a number of cylinders 12 matching the number of the applications required, each already equipped with an internal pushing piston 13, a single actuating rod 14 and a tube or container 16 holding a medicinal ointment 30; alternatively, the confection 40 may contain a series of medical cylinders 12 already filled by the manufacturer of the ointment with an appropriate volume of medicinal product 30, and whose extremities will be sealed off by one or two caps; the confection 40 may at any rate include a single actuating rod 14, protected during the various applications of the medicinal product 30 by not being in direct contact with the ointment, and therefore of a clearly re-usable type.

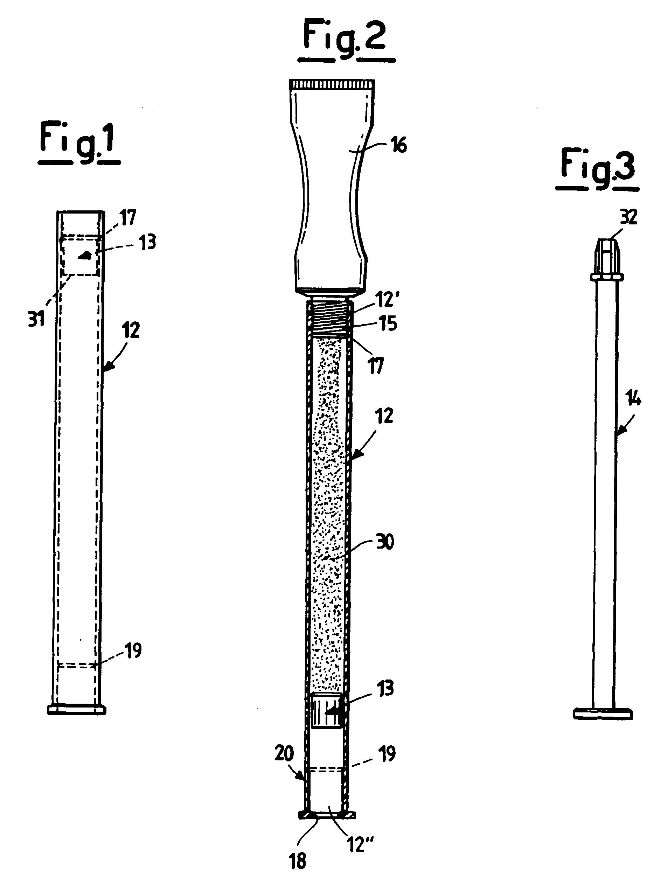

At first and as shown in Figure 1, only the cylinder 12 already fitted with an internal pushing piston 13 is used.

In the following, if the confection includes a series of 12 empty cylinders and a single container 16 of medicinal ointments 30, the container 16 is threaded onto the threaded portion 15 of the cylinder 12. This allows the medicinal ointment 30 to be admitted to the cylinder 12 of the cannula 11 by pressing the container 16.

In this phase the flowing ointment 30 exiting from the container 16 gradually pushes the piston 13, which is initially resting against the undercut 17 of the extremity 12', until it stops against the shoulder 19.

The motion of the small piston 13 inside the cylinder 12 is facilitated by the fact that it is first of all manufactured from a low-friction sliding material, and next, that the presence of some micro-slits 20 favors a practically complete filling of the cylinder 12, accompanied by an appropriate venting of air.

In the following, in order to apply the medicinal ointment 30 it suffices to actuate the piston 13 by inserting the rod 14 and pushing it forward, which happens in this case to be detachably connected to the piston 13, when coming into contact, after introducing it into the hole 18 of the cylinder 12, with the lower surface 31 facing the annular shoulder 22.

The extremity 32 of the actuating rod 14 is brought in contact with the surface 31 of the small piston 13 and every forward sliding action on the part of an external user turns into a pushing action on the piston 13 to make it move inside the cylinder 12, so as to compress the medicinal product for the desired application 30 and force it to flow from the threaded extremity 12' to the outside, obviously after the small tube 16 has been unscrewed from the cylinder 12.

It should be noted that the rod 14 may assume an extremely simple shape (a small elongated cylinder) so as to further minimize the production costs, because it suffices to have the terminal surface 32 of the rod 14 interact in some way with the lower surface 31 of the piston 13 in order to push the medicinal product 30 toward the outside.

In an alternative but non-limiting embodiment of the cannula 11 according to this invention, an actuating rod 14' may be fitted with a profiled extremity 23, capable of coupling more effectively with the surface 31 of the piston 13.

In this case the extremity 23 of the rod 14' preferably includes a pair of discs 24 capable of interacting with the internal walls of the cylinder 12; in a corresponding way, a portion of the lateral surface of the cylinder 12, arranged in an area next to the extremity 12'', is fitted with a profiled slit 25 profiled to follow that of the portion 23 of the rod 14, through which the rod 14 may be introduced and/or extracted in a lateral sense and actuated inside the cylinder 12.

A further exemplifying but non-limiting embodiment of this invention covers a cannula 11 already filled with an amount of medicinal ointment 30 and ready to use, sealed by a cap which is detachably connected to the threaded extremity 12' of the cylinder 12.

The pushing cylinder 13 happens to be initially held against the shoulder 19.

In order to use this cannula 11, it merely suffices to remove the cap and an eventual further cap similar to the previous one and inserted next to the hole 18, and therefore to insert the actuating rod 14 into the hole 18, before starting to move the piston 13.

The above description clearly outlines the characteristics of the dispensing tube for medicinal ointments as an object of this invention, and also clarifies its advantages.

In particular, these are represented by:
- an easy filling of the cannulas,
- a reduced wastage of material,
- a possibility to reutilize the same actuating rod for multiple applications of the medicinal product,
- a maintenance of hygienic conditions,
- limited overall production costs, with respect to those of traditional cannulas.

It is finally obvious that numerous other variations may be applied to the cannula for medicinal ointments as an object of this invention, without thereby abandoning the principles of novelty inherent in the inventive idea, just as it is obvious that in the practical implementation of the invention the materials, the shapes and dimensions of the described details may be of any kind depending on the requirements, and that the same may be substituted by others of a technically equivalent kind.

## Claims

1. A cannula (11) for medicinal ointments comprising a cylinder (12) capable of holding said medicinal ointment (30) and a pushing piston (13) moved by a rod (14, 14') inside said tube or cylinder (12), characterized in that said rod (14, 14') is detachably connected to said piston (13).

2. A cannula (11) according to claim 1, characterized in that said detachable connection is produced by bringing at least one surface portion (32) of the extremity of said rod (14, 14') in contact with at least one surface portion of the extremity of said pushing piston (13), so as to allow said rod (14, 14') to push said piston (13) to slide inside said cylinder (12).

3. A cannula (11) according to claim 2, characterized in that said rod (14) can be inserted inside said cylinder (12) through a hole (18) positioned opposite said surface portion of said piston (13).

4. A cannula (11) according to claim 1, characterized in that it provides, next to a least one first extremity (12') of said cylinder (12), an undercut (17) acting as a shoulder to rest the said piston (13).

5. A cannula (11) according to claim 3, characterized in that it provides, next to said first extremity (12') of said cylinder (12), a threaded portion (15) which may be associated with a container (16) of medicinal products or at least with a cap.

6. A cannula (11) according to claim 1, characterized in that said cylinder (12) provides, next to at least one second extremity (12''), a greater internal diameter size than that of its other portions, and a bottom shoulder (19) set essentially at an area of change of said diameter, where said piston (13) comes to rest at the end of a filling operation of said cannula (11).

7. A cannula (11) according to claim 1, characterized in that said cylinder (12) is fitted with some air venting slits (20).

8. A cannula (11) according to claim 6, characterized in that said rod (14') has at least one profiled extremity (23) and that said cylinder (12) is correspondingly fitted with at least one profiled slit (25) provided next to said second extremity (12'') of said cylinder (12), through which said rod (14, 14') may be introduced and/or extracted from said cylinder (12).

9. A cannula (11) according to claim 1, characterized in that said ointments or medicinal products are constituted by vaginal and/or anal ointments.
